(19) Europäisches Patentamt / European Patent Office / Office européen des brevets

(11) **EP 4 473 307 B1**

(12) **EUROPÄISCHE PATENTSCHRIFT**

(45) Veröffentlichungstag und Bekanntmachung des Hinweises auf die Patenterteilung:
**16.04.2025 Patentblatt 2025/16**

(21) Anmeldenummer: **23702027.6**

(22) Anmeldetag: **31.01.2023**

(51) Internationale Patentklassifikation (IPC):
***G01N 33/18*** *(2006.01)*

(52) Gemeinsame Patentklassifikation (CPC):
**G01N 33/1886;** G01N 27/4166

(86) Internationale Anmeldenummer:
**PCT/EP2023/052219**

(87) Internationale Veröffentlichungsnummer:
**WO 2023/148130 (10.08.2023 Gazette 2023/32)**

(54) **VERFAHREN ZUM STROMSPARENDEN FORTLAUFENDEN MESSEN EINER QUALITÄT EINER FLÜSSIGKEIT UND MESSVORRICHTUNG ZUR DURCHFÜHRUNG DES VERFAHRENS**

ENERGY-EFFICIENT METHOD FOR CONTINUOUSLY MEASURING THE QUALITY OF A LIQUID AND MEASURING DEVICE FOR CARRYING OUT THE METHOD

PROCÉDÉ DE MESURE EN CONTINU À FAIBLE CONSOMMATION DE COURANT D'UNE QUALITÉ D'UN LIQUIDE ET DISPOSITIF DE MESURE DESTINÉ À LA MISE EN ŒUVRE DU PROCÉDÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **01.02.2022 EP 22154550**

(43) Veröffentlichungstag der Anmeldung:
**11.12.2024 Patentblatt 2024/50**

(73) Patentinhaber: **Badger Meter Austria GmbH
1030 Wien (AT)**

(72) Erfinder:
• **MORAWEK, Roman
1200 Wien (AT)**
• **HASELBERGER, Christian
1200 Wien (AT)**

(74) Vertreter: **SONN Patentanwälte GmbH & Co KG
Riemergasse 14
1010 Wien (AT)**

(56) Entgegenhaltungen:
JP-A- S59 162 444      US-A- 4 464 230
US-A1- 2009 068 754

**Beschreibung**

Die Erfindung betrifft ein Verfahren zum stromsparenden fortlaufenden Messen einer Qualität einer Flüssigkeit, insbesondere Wasser, mit einem Sensor zur Erfassung einer Messgröße.

Zudem betrifft die Erfindung eine Messvorrichtung zum stromsparenden fortlaufenden Messen einer Qualität einer Flüssigkeit, insbesondere von Wasser, mit einem Sensor.

Bekannter Weise wird die Qualität von Flüssigkeit, beispielsweise Wasser, mit Sensoren erfasst, wobei die Sensoren mit elektrischer Energie versorgt werden. Steht hierfür keine externe Stromversorgung dauerhaft zur Verfügung, wird die Energie für die Sensoren mittels elektrischer Energiespeicher, bspw. Batterien, insbesondere wiederaufladbarer Batterien, bereitgestellt. Solche elektrische Energiespeicher werden im Zuge von Wartungstätigkeiten erneuert oder wieder aufgeladen, was jedoch mit einem entsprechenden Wartungsaufwand verbunden ist. Daher ist es wünschenswert, einen geladenen Zustand der Energiespeicher möglichst lange aufrecht zu erhalten, d.h. die Sensoren möglichst stromsparend zu betreiben. Dies wird oftmals durch ein verlängertes Messintervall, d.h. durch größere Zeitabstände zwischen aufeinanderfolgenden Messungen mit dem Sensor erzielt. Zwischen den Messungen wird der Sensor deaktiviert und eine Steuerung für den Sensor wird in einen Ruhe- bzw. Schlafzustand versetzt. Nach Ablauf einer definierten Zeit verlässt die Steuerung den Ruhezustand und aktiviert den Sensor für kurze Zeit, um die Messung auszuführen.

Während ein solcher stromsparender Betrieb für manche Sensoren durchaus zweckmäßig ist, erfordern andere Sensoren zur Messung einer Qualität von Flüssigkeit, insbesondere Wasser, eine im Wesentlichen durchgehende Stromversorgung, zumindest jedoch eine vergleichsweise wesentlich längere Stromversorgung, bspw. von mehreren Stunden, um korrekte Messergebnisse zu liefern. Würden diese anderen Sensoren für mehrere Stunden mit Strom versorgt, um ein einziges Messergebnis zu erhalten, und dann deaktiviert, um Energie zu sparen, erhält man mit diesen anderen Sensoren nur wenige Messergebnisse pro Tag. Eine derart geringe Messrate ist jedoch zur Messung einer Qualität von Flüssigkeit, insbesondere Wasser, oftmals unzureichend.

Wie in AT 520515 A1 ausgeführt, sind Sensoren zur Messung der Wasserqualität manchmal auch an Pumpen angebunden, welche den Wassertransport zum Sensor sicherstellen. In solchen Systemen muss zum Ausführen von Messungen daher neben dem Sensor selbst auch die zugeordnete Pumpe aktiviert werden, was den gesamten Energiebedarf weiter erhöht. Abgesehen von einer kurzen Vorlaufzeit zur Sicherstellung des Wasseraustauschs vor Sensoraktivierung, kann in der stromlosen Phase des Sensors auch die Pumpe stromlos geschaltet werden, was die Energieeinsparung weiter erhöht.

Die EP 3 194 908 B1 betrifft Überwachungssysteme bspw. für Leitungs- oder Rohrnetze. Das Überwachungssystem kann eine drahtlose Telemetrieeinheit umfassen, die mit mindestens einem Schwellenwertanzeiger zur Erfassung eines Flüssigkeitsstands und mit mindestens einem Sensor verbunden ist, um einen Zustand der Flüssigkeit in einer Leitung des Rohrnetzes zu bestimmen. Die Telemetrieeinheit kann den Sensor mit Strom versorgen, wenn der Schwellenwertanzeiger einen Flüssigkeitsstand oberhalb eines Schwellenwerts anzeigt. Ebenso kann die Telemetrieeinheit die Stromzufuhr zum Sensor unterbrechen, wenn der Schwellenwertanzeiger einen Flüssigkeitsstand unter dem Schwellenwert anzeigt. Auf diese Weise wird der Stromverbrauch durch den Sensor gesenkt. Die Druckschrift erwähnt bereits, dass der Sensor, sobald er mit Strom versorgt wird, eine gewisse Zeit zum Aufwärmen benötigt. Details zur Aufwärmphase oder Lösungen zur Umgehung der Aufwärmzeit sind nicht angegeben.

Die US 2020/0340968 A1 betrifft eine Vorrichtung zur Überwachung von Parametern von Flüssigkeiten, wie z.B. Wasserqualität, auf energiesparende Weise. Die Vorrichtung umfasst bspw. einen Chlorsensor und eine Steuerung die den Betrieb der Vorrichtung zumindest zwischen einem ausgeschalteten Modus, einem aktiven Modus, in welchem die Vorrichtung Messungen durchführt, und einem Ruhemodus steuert, in welchem keine Messungen durchgeführt werden und die Steuerung und der Chlorsensor nur minimal mit Strom versorgt werden, sodass der Chlorsensor nicht in einen passiven Modus übergeht und nicht neu kalibriert werden muss. Die hierfür erforderliche geringe Energie kann aus Batterien entnommen werden. Die US 2020/0340968 A1 beschreibt somit einen Stromsparbetrieb eines Chlorsensors, nicht jedoch ein Ausschalten des Sensors zwischen aufeinanderfolgenden Messungen und wie damit verbundene Messungenauigkeiten verhindert werden.

Die JP S59 162444 A offenbart eine Vorrichtung und ein Verfahren zum Messen der Alterung von Öl, bspw. in Kraftfahrzeugen. Hierfür wird zunächst die Öltemperatur gemessen und wenn diese in einem definierten Temperaturbereich liegt, ein Spannungsimpuls an eine im Öl angeordnete Elektrode angelegt. Die zeitliche Änderung des infolge des Spannungsimpulses durch das Öl fließenden Stroms wird gemessen und daraus auf die Ölalterung geschlossen. Die US 4 464 230 A offenbart ein Verfahren zum Erfassen eines Sauerstoffgehalts in einem Fluidstrom, bspw. Wasser. Hierfür wird eine Elektrode in das Wasser eingebracht, für eine erste Zeitdauer aktiviert, und für ein Zeitintervall deaktiviert, das länger als die aktivierte Zeitdauer ist.

Die US 2009/068754 A1 betrifft ein Biosensorsystem, welches eine Analytkonzentration bspw. von Alkohol, Glukose, Harnsäure, Laktat, Cholesterin, Bilirubin und dergleichen einer biologischen Probe aus einem Ausgangssignal mit einem abklingenden Verlauf bestimmt. Ein Sensorstreifen, welcher die zu messende Probe aufnimmt, wird hierfür mit einem oder mehreren Impulsen mit Strom angeregt.

Die Aufgabe der Erfindung besteht in der Schaffung eines Verfahrens und einer Messvorrichtung der eingangs genannten Art, welche eine stromsparende fortlaufende Messung einer Qualität einer Flüssigkeit, insbesondere Wasser, mit einem Sensor ermöglichen. Insbesondere soll der Sensor im Vergleich zu einem üblichen Betrieb mit geringerem Stromverbrauch betrieben werden, wobei dennoch Messergebnisse mit im Wesentlichen gleicher Genauigkeit wie im Fall des üblichen Betriebs mit höherem Stromverbrauch erhalten werden.

Hierfür sieht die Erfindung ein Verfahren wie in Anspruch 1 und eine Messvorrichtung wie in Anspruch 12 definiert vor. Vorteilhafte Ausführungsformen und Weiterbildungen sind in den abhängigen Ansprüchen angegeben.

Die Aufgabe wird erfindungsgemäß durch ein Verfahren zum stromsparenden fortlaufenden Messen einer Qualität einer Flüssigkeit, insbesondere Wasser, mit einem Sensor zur Erfassung einer Messgröße gelöst, wobei:

S1) der Sensor als Teil einer Messvorrichtung zur Messung der Flüssigkeitsqualität bereitgestellt und die Messvorrichtung relativ zur Flüssigkeit angeordnet wird;

S2) der Sensor abwechselnd für die Dauer einer Einschaltphase aktiviert und für die Dauer einer Ausschaltphase deaktiviert wird;S3)eine Einschaltphase in einem eingeschwungenen Zustand des Sensors als definierte Einschaltphase festgelegt wird und mit dem Sensor während der definierten Einschaltphase ein Messwert als Vergleichsmesswert erfasst wird, wobei der eingeschwungene Zustand erreicht ist, wenn sich ein Wert der Messgröße um höchstens eine vorgegebene Differenz von einem Wert der Messgröße während einer vorherigen Einschaltphase unterscheidet;

S4) eine Referenzmessung zur Erfassung eines die Flüssigkeitsqualität repräsentierenden Messwerts, als Referenzmesswert ausgeführt wird;

S5) mit dem Sensor während weiterer Einschaltphasen, welche auf die definierte Einschaltphase folgen, laufend Messwerte als Betriebsmesswerte erfasst werden;

S6) eine Berechnungsvorschrift festgelegt wird, welche den Vergleichsmesswert dem Referenzmesswert zuordnet; und

S7) mittels der Berechnungsvorschrift aus jedem Betriebsmesswert ein finaler Messwert zur Messung der Qualität ermittelt wird; wobei

für die Ausführung der Referenzmessung

S4b) die definierte Einschaltphase oder eine Einschaltphase zwischen der definierten Einschaltphase und den weiteren Einschaltphasen zur Erfassung der Betriebsmesswerte gegenüber den vorangegangenen Einschaltphasen verlängert wird, und während der verlängerten Einschaltphase der Referenzmesswert mit dem Sensor erfasst wird.

Der Sensor wird somit in einem Schritt S1 als Teil einer Messvorrichtung zur Messung der Flüssigkeitsqualität bereitgestellt und die Messvorrichtung wird relativ zur Flüssigkeit angeordnet. Dabei wird der Sensor in der Flüssigkeit angeordnet. Der den Sensor nicht enthaltende Teil der Messvorrichtung kann zumindest teilweise in der Flüssigkeit oder in einem Abstand von der Flüssigkeit angeordnet werden. Bspw. ist der den Sensor nicht enthaltende Teil der Messvorrichtung über ein elektrisch leitendes Kabel mit dem Sensor verbunden. Alternativ kann der Sensor in die Messvorrichtung eingesetzt sein.

In einem Schritt S2 wird der Sensor abwechselnd für die Dauer einer Einschaltphase aktiviert und für die Dauer einer Ausschaltphase deaktiviert. Im aktivierten Zustand wird der Sensor mit elektrischem Strom versorgt und im deaktivierten Zustand nicht mit elektrischem Strom versorgt. Hierdurch wird der Sensor stromsparend betrieben und das Verfahren stromsparend durchgeführt. Die Dauer der Einschaltphase und die Dauer der Ausschaltphase können von einem Betreiber der Messvorrichtung eingestellt werden.

In einem Schritt S3 wird mit dem Sensor während einer definierten Einschaltphase ein Messwert als Vergleichsmesswert erfasst. In der definierten Einschaltphase unterscheidet sich der Vergleichsmesswert um höchstens eine vorgegebene Differenz von einem während einer vorherigen Einschaltphase erfassbaren Wert der Messgröße. Es sei darauf hingewiesen, dass sich der mit dem Sensor erfassbare Wert der Messgröße, selbst bei konstanter Messgröße, wegen des abwechselnden Aktivierens und Deaktivierens des Sensors während der Einschaltphasen ändert. Wenn daher im weiteren Verlauf der Beschreibung auf eine Erfassung mehrerer Messwerte oder einen Vergleich mehrerer Messwerte Bezug genommen wird, so ist dies selbstverständlich so zu verstehen, dass die Messwerte zu ähnlichen, insbesondere gleichen Zeitabständen vom Beginn der jeweiligen Einschaltphase erfasst bzw. betrachtet werden, um korrekte und vergleichbare Messergebnisse zu erhalten. Somit werden für einen korrekten Vergleich der Vergleichsmesswert und der Wert der Messgröße einer vorherigen Einschaltphase bevorzugt in gleichen Zeitabständen vom Beginn der jeweiligen Einschaltphase erfasst bzw. betrachtet. Hinsichtlich der Einschaltphase kann von einem Betreiber der Messvorrichtung festgelegt werden, welche Einschaltphase die definierte Einschaltphase ist. Der Vergleichsmesswert wird somit am Ende oder nach einer Einschwingphase des abwechselnd aktivierten und deaktivierten Sensors erfasst, wobei dann die Werte der Messgröße in gleichen Abständen vom Beginn der Einschaltphasen im Wesentlichen stabil sind, d.h. sich um nicht mehr als die vorgegebene Differenz voneinander unterscheiden. Diese Einschwingphase liegt vor, wenn der Sensor nach einer im Vergleich zur Dauer der Ausschaltphase verlängerten Ausschaltphase, die bspw. zu Wartungszwecken erforderlich war, oder nach einer erstmaligen Aktivierung des Sensors abwechselnd aktiviert und deaktiviert wird. Während der Einschwingphase werden sich die Werte der Messgröße in mehreren aufeinanderfolgenden Einschaltphasen und in gleichen Abständen vom Beginn der Einschaltphasen betrachtet zunächst unterscheiden, sich jedoch zunehmend an den Vergleichsmesswert annähern. Somit wird in Schritt S3 ein für die Dauer einer Einschaltphase des eingeschwungenen Sensors repräsentativer Vergleichsmesswert erfasst.

In einem Schritt S4 wird eine Referenzmessung zur Erfassung eines die Flüssigkeitsqualität repräsentierenden Messwerts als Referenzmesswert ausgeführt. Die Referenzmessung erfasst den Messwert mit einer Genauigkeit, die bspw. oder annähernd auch mit dem Sensor erzielbar wäre, wenn dieser dauerhaft aktiviert wäre.

In einem Schritt S5 werden mit dem Sensor während weiterer Einschaltphasen, welche auf die definierte Einschaltphase folgen, laufend Messwerte als Betriebsmesswerte erfasst. Die Erfassung der Betriebsmesswerte entspricht dabei dem fortlaufenden Messen einer Qualität der Flüssigkeit, insbesondere Wasser, und wird spätestens durch eine Wartungsaktivität eines Betreibers der Messvorrichtung, bspw. für einen Batterietausch, unterbrochen. Bevorzugt werden die Betriebsmesswerte für eine korrekte Messung in gleichen Abständen vom Beginn der Einschaltphasen erfasst.

In einem Schritt S6 wird eine Berechnungsvorschrift festgelegt, welche den Vergleichsmesswert dem Referenzmesswert zuordnet. Die Berechnungsvorschrift kann bspw. in einer Steuerung der Messvorrichtung ermittelt und in einem Speicher der Messvorrichtung abgelegt werden.

In einem Schritt S7 wird mittels der Berechnungsvorschrift aus jedem Betriebsmesswert ein finaler Messwert zur Messung der Qualität der Flüssigkeit ermittelt. Der finale Messwert repräsentiert somit die Qualität der Flüssigkeit, insbesondere Wasser.

In einem Schritt S4b wird die definierte Einschaltphase oder eine Einschaltphase zwischen der definierten Einschaltphase und den weiteren Einschaltphasen zur Erfassung der Betriebsmesswerte gegenüber den vorangegangenen Einschaltphasen verlängert, und während der verlängerten Einschaltphase wird der Referenzmesswert mit dem Sensor erfasst. Die Referenzmessung wird somit im Vergleich zur Messung während einer Einschaltphase mit höherer Genauigkeit durchgeführt. Die Referenzmessung erfolgt während einer verlängerten Einschaltphase mit dem Sensor selbst. Wenn hierfür die definierte Einschaltphase verlängert wird, erfolgt die Referenzmessung in der definierten Einschaltphase nach der Erfassung des Vergleichsmesswerts.

Das Verfahren sieht somit vor, den Sensor nicht wie üblich dauerhaft mit Strom zu versorgen, sondern abwechselnd zu aktivieren und deaktivieren, um Energie zu sparen und einen Energiespeicher zur Stromversorgung möglichst selten tauschen bzw. wieder aufladen zu müssen. Nach einer Einschwingphase des Sensors wird in einer vergleichsweise kurzen Einschaltphase ein Vergleichsmesswert mit geringerer Genauigkeit erfasst und einem Referenzmesswert mit höherer Genauigkeit zugeordnet. Im fortlaufenden Messbetrieb werden während weiterer vergleichsweise kurzer Einschaltphasen Betriebsmesswerte mit geringerer Genauigkeit erfasst. Schließlich werden mit Hilfe des Zusammenhangs zwischen dem Vergleichsmesswert und dem Referenzmesswert die Ungenauigkeiten der Betriebsmesswerte korrigiert, wodurch finale Messwerte mit höherer Genauigkeit erhalten werden.

Wenn der Sensor zumindest zeitweise periodisch aktiviert und deaktiviert wird, kann die Genauigkeit des Vergleichsmesswerts und/oder der Betriebsmesswerte verbessert werden. Dies kann damit begründet werden, dass die Messwerte des Sensors nicht nur vom Wert der Messgröße selbst, sondern auch von der Dauer der vorangegangenen Einschaltphasen und Ausschaltphasen abhängen können. Ständig variierende Dauern der Einschaltphasen und Ausschaltphasen können daher auch bei einem gleichbleibenden Wert der Messgröße zu variierenden Messwerten führen. Besonders bevorzugt wird der Sensor während des laufenden Messbetriebs, nach der definierten Einschaltphase, zumindest zeitweise periodisch aktiviert und deaktiviert.

Betreffend die definierte Einschaltphase kann vorgesehen sein, dass
S3a) eine Einschaltphase zumindest dann als definierte Einschaltphase festgelegt wird, wenn eine festgelegte Anzahl von vorangehenden Einschaltphasen auf eine im Vergleich zur Dauer der Ausschaltphase verlängerte Ausschaltphase oder eine erstmalige Aktivierung des Sensors folgen.

Die Anzahl von vorangehenden Einschaltphasen kann einstellbar sein. Beispielsweise kann ein Betreiber der Messvorrichtung diese Anzahl auf Grundlage von Erfahrungswerten festlegen. Die Anzahl von vorangehenden Einschaltphasen und somit auch vorangehenden Ausschaltphasen kann derart festgelegt werden, dass sich der Sensor danach zuverlässig im eingeschwungenen Zustand befindet. Gezählt wird die festgelegte Anzahl von vorangehenden Einschaltphasen von einem Zeitpunkt des erstmaligen Einschaltens des Sensors oder im Anschluss an eine längere Betriebsunterbrechung des Sensors, d.h. an eine Ausschaltphase, die im Vergleich zur Dauer vorangegangener Ausschaltphasen oder zu einer durchschnittlichen Dauer vorangegangener Ausschaltphasen länger ist. Beispielsweise kann eine durchschnittliche Ausschaltphase 20 Minuten betragen, während eine Betriebsunterbrechung 1 Stunde betragen kann.

Betreffend die definierte Einschaltphase kann weiters vorgesehen sein, dass

S3b) eine Einschaltphase zumindest durch folgendes als definierte Einschaltphase festgelegt wird:

- Erfassen von Messwerten mit dem Sensor in mehreren aufeinander folgenden Einschaltphasen;
- Vergleichen des zuletzt erfassten Messwerts mit dem davor erfassten Messwert; und
- im Falle einer einen vorab definierten Schwellenwert unterschreitenden Abweichung der beiden verglichenen Messwerte voneinander, Festlegen der Einschaltphase für den letzten Messwert als die definierte Einschaltphase.

Hierdurch kann jene Einschaltphase als definierte Einschaltphase festgelegt werden, für welche sich der Messwert um weniger als den definierten Schwellenwert vom vorherigen Messwert unterscheidet. Der Sensor befindet sich somit im eingeschwungenen Zustand. Für einen korrekten Vergleich der Messwerte werden die Messwerte bevorzugt in gleichen Zeitabständen vom Beginn der jeweiligen Einschaltphase erfasst.

Weiters ist es günstig, wenn der Vergleichsmesswert und/oder der Betriebsmesswert durch Messen des Vergleichsmesswerts und/oder des Betriebsmesswerts zu einem festgelegten Soll-Zeitpunkt innerhalb der Einschaltphase, insbesondere am Ende der Einschaltphase, erfasst werden. Auf diese Weise können besonders zuverlässige Messergebnisse erzielt werden, da sich, wie zuvor bereits erwähnt, selbst bei konstanter Messgröße der mit dem Sensor erfassbare Wert der Messgröße wegen des abwechselnden Aktivierens und Deaktivierens des Sensors während der Einschaltphasen ändert. Der Soll-Zeitpunkt kann bspw. von einem Betreiber der Messvorrichtung festgelegt werden und einstellbar sein. Der Soll-Zeitpunkt kann, muss aber nicht am Ende der Einschaltphase liegen.

Weiters kann vorgesehen sein, dass der Vergleichsmesswert und/oder der Betriebsmesswert durch folgendes erfasst werden:

- Messen mit dem Sensor zu mehreren Zeitpunkten innerhalb der dem Vergleichsmesswert und/oder Betriebsmesswert zugeordneten Einschaltphase, um mehrere Abtastwerte zu erhalten;
- Berechnen eines an die Abtastwerte angenäherten Messwertverlaufs innerhalb der Einschaltphase;
- Messen zu welchem Ist-Zeitpunkt der für die Messung zu einem definierten Soll-Zeitpunkt vorgesehene Vergleichsmesswert und/oder Betriebsmesswert tatsächlich gemessen wurde; und
- im Falle einer Abweichung des Ist-Zeitpunkts vom Soll-Zeitpunkt, Berechnen des Vergleichsmesswerts und/oder Betriebsmesswerts zum Soll-Zeitpunkt mittels des angenäherten Messwertverlaufs.

Auf diese Weise können Fehler bei der Erfassung des Vergleichsmesswerts und/oder Betriebsmesswerts korrigiert werden, welche Fehler dadurch entstehen können, dass der beabsichtigte Messzeitpunkt, d.h. der Soll-Zeitpunkt, nicht eingehalten werden kann. Bspw. kann der Soll-Zeitpunkt wegen systembedingter Ungenauigkeiten, wie einer vorübergehenden Überlastung eines Prozessors der Messvorrichtung, nicht eingehalten werden. Aus der Kenntnis des Messwertverlaufs innerhalb der Einschaltphase kann der Messwert zum vorgesehenen Soll-Zeitpunkt aus dem zum Ist-Zeitpunkt erfassten Messwert, vorzugsweise durch die Messvorrichtung selbst, berechnet werden, insbesondere durch Interpolation oder Extrapolation. Dabei kann der Soll-Zeitpunkt zeitlich vor oder nach dem Ist-Zeitpunkt liegen.

Eine besonders einfache Ausführungsform kann vorsehen, dass

- die Berechnungsvorschrift als Berechnen eines konstanten Faktors, welcher aus dem Quotienten zwischen dem Vergleichsmesswert und dem Referenzmesswert gebildet wird, festgelegt wird und
- der finale Messwert aus jedem Betriebsmesswert durch Dividieren des Betriebsmesswerts durch den berechneten konstanten Faktor ermittelt wird.

Es gilt demnach

$$V/R = B/F = Q,$$

$$F = B/Q$$

wobei V der Vergleichsmesswert, R der Referenzmesswert, B ein Betriebsmesswert, F ein finaler Messwert und Q der konstante Faktor ist.

Die durch diese Gleichungen berechneten finalen Messwerte werden den tatsächlichen Wert der Messgröße sehr gut annähern, solange die Betriebsmesswerte nur geringfügig vom Vergleichsmesswert abweichen. Mit zunehmender Abweichung, bspw. in Folge einer Veränderung der Flüssigkeitsqualität, wird diese einfache Berechnungsvorschrift wegen des nicht linearen Verlaufs der Messgröße in der Einschaltphase ungenauer.

Um möglichst korrekte finale Messwerte zu erzielen ist es günstig, wenn

S8) die Schritte S2) bis S7) erneut durchgeführt werden, wenn mehrere miteinander verglichene Betriebsmesswerte oder mehrere miteinander verglichene finale Messwerte um mehr als einen vorab definierten Schwellenwert voneinander abweichen.

Eine solche Abweichung mehrerer miteinander verglichener Betriebsmesswerte oder finaler Messwerte ist ein Hinweis auf eine sich verändernde Qualität der Flüssigkeit. Demnach sind auch ein geänderter Vergleichsmesswert und ein geänderter Referenzmesswert erwartbar, weshalb die Schritte S2) bis S7) des Verfahrens bevorzugt erneut durchgeführt werden. Der vorab definierte Schwellenwert kann bspw. von einem Betreiber der Messvorrichtung eingestellt werden. Bevorzugt werden zuletzt gemessene Betriebsmesswerte oder zuletzt berechnete finale Messwerte mit länger zurückliegenden Betriebsmesswerten oder finalen Messwerten verglichen.

Des weiteren wird bevorzugt, dass S9) zumindest die Schritte S2), S3) und S5) bis S7) erneut durchgeführt werden, wenn die Dauer der Einschaltphase und/oder die Dauer der Ausschaltphase um mehr als einen vorab definierten Schwellenwert geändert wurde. Die Änderung der Dauer der Einschaltphase und/oder der Dauer der Ausschaltphase kann von einem Betreiber der Messvorrichtung durchgeführt werden, bspw. um finale Messwerte in kürzeren Zeitabständen zu erhalten oder die Stromeinsparung durch den Sensor noch weiter zu erhöhen. Da die mit dem Sensor erfassbaren Messwerte auch von der Dauer der Einschaltphase und/oder der Dauer der Ausschaltphase abhängen, ist auch ein geänderter Vergleichsmesswert erwartbar, weshalb zumindest die Schritte S2), S3) und S5) bis S7) des Verfahrens bevorzugt erneut durchgeführt werden. Wenn der Vorgang der Referenzmessung von den geänderten Einschaltphasen/Ausschaltphasen unabhängig ist und somit kein neuer Referenzmesswert erwartbar ist, kann der Schritt S4) der erneuten Referenzmessung auch entfallen.

Um korrekte finale Messwerte zu erhalten ist es zweckmäßig, wenn die Schritte S2) bis S5) mit der gleichen Flüssigkeit durchgeführt werden.

Zur Erzielung korrekter finaler Messwerte mit Messvorrichtungen, in welchen eine Flüssigkeitszufuhr zum Sensor durch eine Flüssigkeitspumpe ausgeführt ist, ist es weiterhin günstig, wenn S10) eine Flüssigkeitspumpe der Messvorrichtung, welche Flüssigkeitspumpe die Flüssigkeit über den Sensor leitet, bereits vor dem Sensor aktiviert wird, beispielsweise eine Minute bis zwei Minuten vor dem Schritt S2) aktiviert wird, insbesondere für einen Zeitraum welcher ausreichend ist, die Flüssigkeit dem Sensor zuzuführen, vor dem Sensor aktiviert wird. Dies ist zweckmäßig, damit die Flüssigkeit am Sensor vorab ausgetauscht wird und unmittelbar zu dessen Einschaltzeitpunkt bereits repräsentativ jener Flüssigkeit entspricht, die an der Entnahmestelle, zum Beispiel in einem Versorgungsrohr, vorliegt.

Besonders bevorzugt wird, dass als Sensor ein Sensor zur Messung der Konzentration eines Desinfektionsmittels, insbesondere zur Messung einer Chlorkonzentration, oder zur Messung eines pH-Werts, verwendet wird. Diese Sensoren erfordern für gewöhnlich lange Aktivierungszeiten, bevorzugt einen durchgehenden Betrieb, um korrekte Messergebnisse zu liefern. Zudem sind diese Sensoren zumindest in Bezug auf wechselnder Qualität der Flüssigkeit, variierenden Dauern der Einschaltphase und Ausschaltphase, der Anzahl vorangegangener Einschaltphasen und Ausschaltphasen und der Verweildauer in der Flüssigkeit besonders empfindlich. Mit dem erfindungsgemäßen Verfahren können auch mit solchen Sensoren auf stromsparende Weise finale Messwerte mit einer Genauigkeit erzielt werden, welche Genauigkeit nur durch kürzere Einschaltphasen allein nicht erzielbar wäre.

Die eingangs gestellte Aufgabe wird erfindungsgemäß zudem durch eine Messvorrichtung zum stromsparenden fortlaufenden Messen einer Qualität einer Flüssigkeit, insbesondere von Wasser, aufweisend zumindest einen Sensor, einen Speicher für elektrische Energie und eine Steuerung gelöst, welche dadurch gekennzeichnet ist, dass die Steuerung zur Durchführung eines Verfahrens gemäß der vorangehenden Beschreibung ausgebildet ist. Die Messvorrichtung weist demnach zumindest einen Sensor auf, mit welchem Werte der die Flüssigkeitsqualität repräsentierenden Messgröße, bspw. eine Konzentration eines Desinfektionsmittels, eine Chlorkonzentration oder ein pH-Wert erfasst werden können. Um die Messvorrichtung unabhängig von einem Stromanschluss betreiben zu können, weist diese den Speicher für elektrische Energie auf. Die Steuerung der Messvorrichtung ist ausgebildet, die Durchführung des zuvor beschriebenen Verfahrens zu steuern. Hierfür weist die Messvorrichtung bevorzugt einen Mikroprozessor und einen Datenspeicher auf. Hinsichtlich der Merkmale der Messvorrichtung wird zudem auf die vorangehende Beschreibung des Verfahrens verwiesen, soweit diese für das Verständnis der Messvorrichtung hilfreich ist und soweit aus dieser Beschreibung des Verfahrens Merkmale der Messvorrichtung ableitbar sind. Ebenso wird hinsichtlich der Merkmale des Verfahrens auch auf die Beschreibung der Messvorrichtung verwiesen.

Besonders bevorzugt ist der Sensor zur Messung der Konzentration eines Desinfektionsmittels, insbesondere zur Messung einer Chlorkonzentration, oder zur Messung eines pH-Werts ausgebildet.

Die Erfindung wird im Folgenden anhand von bevorzugten, nicht einschränkenden Ausführungsbeispielen unter Bezugnahme auf die Zeichnung noch weiter erläutert. Es zeigen:

Fig. 1 eine schematische Darstellung eines Teils des zeitlichen Ablaufs des Verfahrens gemäß der Erfindung;

Fig. 2 eine schematische Darstellung des zeitlichen Ablaufs einer Einschaltphase in größerem Maßstab;

Fig. 3 ein Ablaufdiagramm zum Verfahren gemäß der Erfindung;

Fig. 4 ein detaillierteres Ablaufdiagramm zum Verfahren gemäß der Erfindung; und

Fig. 5 ein vereinfachtes Blockschaltbild der Messvorrichtung gemäß der Erfindung.

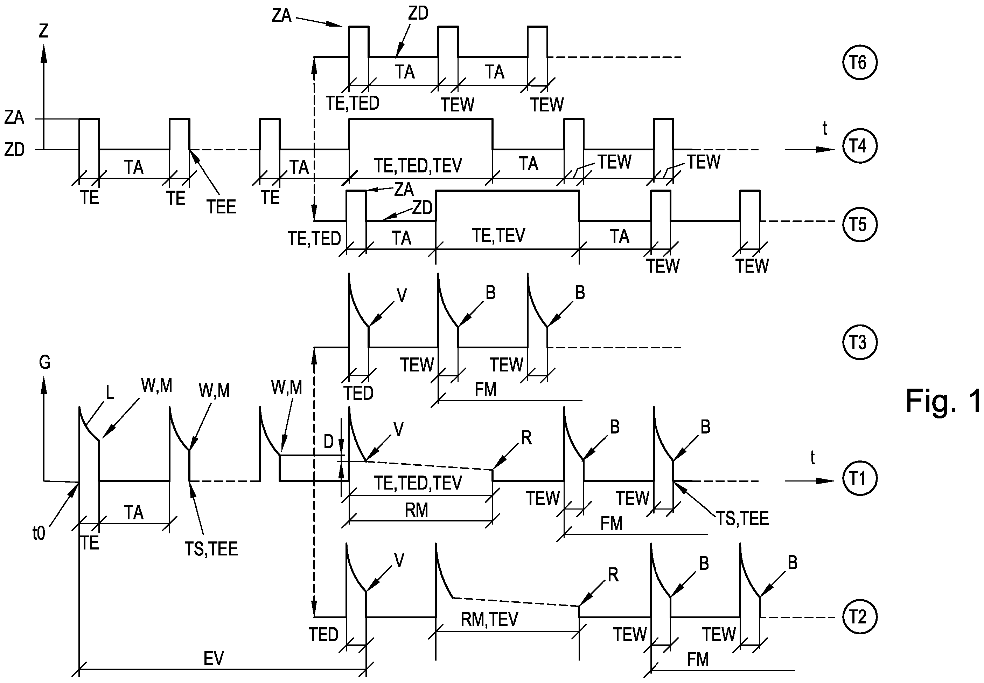

Fig. 1 zeigt, nicht maßstabsgetreu, einen Teil des zeitlichen Ablaufs des Verfahrens zum stromsparenden fortlaufenden Messen FM einer Qualität einer Flüssigkeit, insbesondere Wasser, in schematischer Darstellung. Vor einem Zeitpunkt t0 wird ein Sensor 1, der Teil einer Messvorrichtung 2 ist und der Erfassung einer die Flüssigkeitsqualität repräsentierenden Messgröße G dient, bereitgestellt und die Messvorrichtung 2 wird relativ zur Flüssigkeit angeordnet (in Fig. 1 nicht dargestellt). Im dargestellten Beispiel wird der Sensor 1 zu einem Zeitpunkt t0 erstmals oder nach einer vergleichsweise langen stromlosen Zeitdauer aktiviert. Der Sensor 1 kann ein Sensor 1a zur Messung der Konzentration eines Desinfektionsmittels, insbesondere zur Messung einer Chlorkonzentration, oder zur Messung eines pH-Werts der Flüssigkeit sein. Auf den Zeitpunkt t0 folgend wird der Sensor 1, 1a abwechselnd für die Dauer einer Einschaltphase TE aktiviert und für die Dauer einer Ausschaltphase TA deaktiviert. In Fig. 1 sind mehrere einander abwechselnde Einschaltphasen TE und Ausschaltphasen TA dargestellt. Während einiger auf den Zeitpunkt t0 folgender Einschaltphasen TE und Ausschaltphasen TA kann der Sensor 1, 1a einschwingen. Die Einschwingphase EV resultiert daraus, dass der Sensor 1, 1a nach seiner Aktivierung (in Fig. 1 zum Zeitpunkt t0) für im Vergleich zur Dauer der Einschaltphase TE lange Zeit betrieben werden muss, bspw. für mehrere Stunden, um korrekte Messergebnisse auszugeben. Gemäß dem Verfahren wird der Sensor 1, 1a jedoch immer nur für die kurze Zeitdauer der Einschaltphase TE aktiviert. Dabei ändert sich der mit dem Sensor 1, 1a erfassbare Wert W der Messgröße G und nähert sich sowohl innerhalb der Einschaltphasen TE als auch mit jeder weiteren Einschaltphase TE dem erst nach entsprechend langer Zeit messbaren korrekten Messwert an, vgl. den Messwertverlauf L während einer Einschaltphase TE und in aufeinanderfolgenden Einschaltphasen TE in Fig. 1. In Fig. 1 ist die Änderung eines Messwerts M von einer Einschaltphase TE zur nächsten, jeweils am Ende der Einschaltphase TE, während der Einschwingphase EV erkennbar. Die Einschwingphase EV erstreckt sich über mehrere, bspw. 5 bis 10, Einschaltphasen TE und Ausschaltphasen TA. Nach der Einschwingphase EV kann mit dem Sensor 1, 1a in jeder Einschaltphase TE bei gleichbleibender Flüssigkeitsqualität der gleiche Messwert M erhalten werden. Das Ende der Einschwingphase EV bildet eine definierte Einschaltphase TED. Während der definierten Einschaltphase TED wird mit dem Sensor 1, 1a ein Messwert M als Vergleichsmesswert V erfasst, wobei sich für die definierte Einschaltphase TED der Vergleichsmesswert V um höchstens eine vorgegebene Differenz D von einem während einer vorherigen Einschaltphase TE erfassbaren Wert W der Messgröße G unterscheidet. Hinsichtlich des während einer vorherigen Einschaltphase TE erfassbaren Werts W der Messgröße G ist es unerheblich, ob der Wert W tatsächlich als Messwert M erfasst wird. Wesentlich ist in diesem Zusammenhang nur, dass sich der Vergleichsmesswert V höchstens um die genannte Differenz D von einem Wert W der Messgröße G einer vorangegangen, bspw. der unmittelbar davor liegenden, Einschaltphase TE unterscheidet. Dies kann auch dadurch erzielt werden, dass eine Anzahl von vorangehenden Einschaltphasen TE festgelegt wird und davon ausgegangen wird, dass sich nach dieser Anzahl von vorangehenden Einschaltphasen TE der Vergleichsmesswert V um höchstens die Differenz D von einem während einer vorherigen Einschaltphase TE erfassbaren Wert W der Messgröße G unterscheidet.

Fg. 1 zeigt weiters, dass eine Referenzmessung RM zur Erfassung eines die Flüssigkeitsqualität repräsentierenden Messwerts M, welcher Referenzmesswert R genannt wird, ausgeführt wird. Die Referenzmessung RM kann auf verschiedene Weise durchgeführt werden, was in Fig. 1 durch die Aufspaltung des Zeitablaufs an der Stelle der vertikalen strichlierten Linie in drei zueinander alternative Zeitabläufe T1, T2, T3 veranschaulicht wird.

Der Zeitablauf T1 in Fig. 1 zeigt eine Ausführung des Verfahrens, gemäß welcher die definierte Einschaltphase TED gegenüber den vorangegangenen Einschaltphasen TE verlängert wird und somit eine verlängerte Einschaltphase TEV darstellt, während welcher der Referenzmesswert R mit dem Sensor 1, 1a erfasst wird. Der Vergleichsmesswert V wird in dieser verlängerten Einschaltphase TEV zu jenem Zeitpunkt erfasst, zu welchem er auch erfasst würde, wenn die definierte Einschaltphase TED nicht verlängert wäre.

Der Zeitablauf T2 in Fig. 1 zeigt eine Ausführung des Verfahrens, gemäß welcher eine Einschaltphase TEV zwischen der definierten Einschaltphase TED und den weiteren Einschaltphasen TEW, welche der Erfassung der Betriebsmesswerte B dienen, verlängert wird. Während der verlängerten Einschaltphase TEV wird der Referenzmesswert R mit dem Sensor 1, 1a erfasst.

Der Zeitablauf T3 in Fig. 1 zeigt eine nicht zum Verfahren zählende Ausführung, gemäß welcher eine Flüssigkeitsprobe von der Flüssigkeit entnommen wird und der Referenzmesswert R der entnommenen Flüssigkeitsprobe in einer zur Messvorrichtung 2 externen Vorrichtung erfasst wird. Die Entnahme der Flüssigkeitsprobe, die Erfassung des Referenzmesswerts R und die externe Vorrichtung sind in Fig. 1 nicht dargestellt.

Unabhängig davon auf welche Weise der Referenzmesswert R erfasst wird, werden mit dem Sensor 1, 1a während weiterer Einschaltphasen TEW, welche auf die definierte Einschaltphase TED folgen, d.h. während der fortlaufenden Messung FM, laufend Messwerte M als Betriebsmesswerte B erfasst. Aus jedem Betriebsmesswert B wird mittels einer festgelegten Berechnungsvorschrift ein finaler Messwert F zur Messung der Qualität ermittelt, wobei die Berechnungsvorschrift den Vergleichsmesswert V dem Referenzmesswert R zuordnet.

Fig. 1 zeigt zudem einen Zeitablauf des Aktivierungszustands Z des Sensors 1, 1a, welcher sich in einem aktivierten Zustand ZA und einem deaktivierten Zustand ZD befinden kann. Dabei ist der Zeitablauf T4 in Fig. 1 dem Zeitablauf T1 aus Fig. 1 zugeordnet, der Zeitablauf T5 in Fig. 1 ist dem Zeitablauf T2 aus Fig. 1 zugeordnet und der Zeitablauf T6 in Fig. 1 ist dem Zeitablauf T3 aus Fig. 1 zugeordnet.

In Fig. 1 ist weiteres angedeutet, dass der Sensor 1, 1a zumindest zeitweise periodisch aktiviert und deaktiviert wird. Der periodische Ablauf kann aufgrund von Einschränkungen im System, bspw. einer vorübergehenden Überlastung der Messvorrichtung, geringfügigen Variationen unterliegen, in welchem Fall der Zeitablauf immer noch als periodisch betrachtet wird. Derartige akzeptable Abweichungen von einer exakten Periodizität können im Bereich mehrerer Sekunden bspw. bis zu 10 Sekunden liegen, wenn bspw. die Dauer der Einschaltphase TE zwischen 2 und 4 Minuten und die Dauer der Ausschaltphase zwischen 10 und 20 Minuten beträgt.

Die definierte Einschaltphase TED kann bspw. als jene Einschaltphase TE festgelegt werden, die vom Zeitpunkt t0 aus betrachtet auf eine festgelegte Anzahl von vorangehenden Einschaltphasen TE folgt. Im Bsp. gemäß Fig. 1 ist diese festgelegte Anzahl zumindest drei. Die definierte Einschaltphase TED kann bspw. auch als jene Einschaltphase TE festgelegt werden, für welche sich ein mit dem Sensor 1, 1a erfasster Messwert M von einem in einer vorangegangenen Einschaltphase TE erfassten Messwert M um weniger als einen vorab definierten Schwellenwert D unterscheidet.

Im in Fig. 1 dargestellten Beispiel wird der Messwert M, insbesondere der Vergleichsmesswert V und/oder der Betriebsmesswert B, am Ende der Einschaltphasen TEE erfasst. Generell aber kann der Soll-Zeitpunkt TS, zu welchem die Messwerte M innerhalb der Einschaltphase TE gemessen werden, vorab festgelegt werden und auch vom Ende der Einschaltphasen TEE abweichen. Beispielsweise zeigt der Zeitablauf T1 in Fig. 1 eine Erfassung des Vergleichsmesswerts V und eine Erfassung des Referenzmesswerts R innerhalb derselben Einschaltphase TE zu unterschiedlichen Zeitpunkten.

Die Einschaltphase TE kann bspw. zwischen 2 und 4 Minuten, bevorzugt zwischen 2,5 und 3,5 Minuten, insbesondere 3 Minuten betragen. Die Ausschaltphase TA kann bspw. zwischen 10 und 20 Minuten, bevorzugt zwischen 13 und 17 Minuten, insbesondere 15 Minuten betragen. Die verlängerte Einschaltphase TEV kann sich bspw. über eine Zeitdauer zwischen 1 und 3 Stunden, bevorzugt zwischen 1,5 und 2,5 Stunden, insbesondere von 2 Stunden erstrecken.

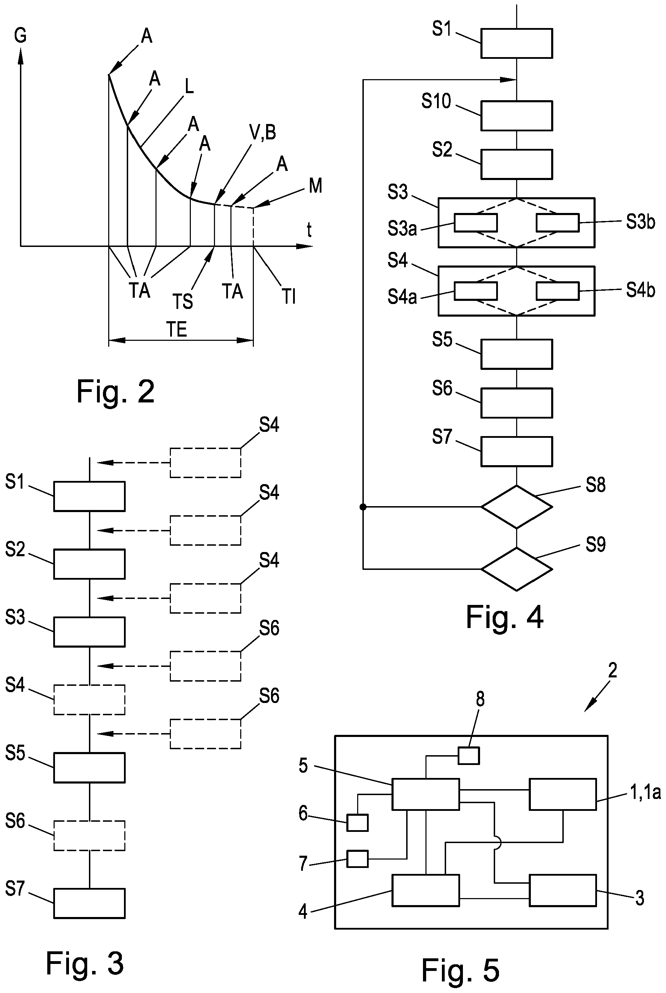

Fig. 2 zeigt beispielhaft eine Erfassung des Vergleichsmesswerts V und/oder eines Betriebsmesswerts B oder allgemein eines Messwerts M mittels eines angenäherten Messwertverlaufs L. Hierfür werden innerhalb der Einschaltphase TE in welcher der Vergleichsmesswert V und/oder der Betriebsmesswert B erfasst werden sollen mit dem Sensor 1, 1a zu mehreren Zeitpunkten TA mehrere Messwerte als Abtastwerte A erfasst. Daraufhin wird ein an die Abtastwerte A angenäherter Messwertverlauf L innerhalb der Einschaltphase TE berechnet. Da die Erfassung des Vergleichsmesswerts V und/oder Betriebsmesswerts B zu einem Soll-Zeitpunkt TS erfolgen soll, wird nun gemessen zu welchem Ist-Zeitpunkt TI der Vergleichsmesswert V und/oder Betriebsmesswert B tatsächlich gemessen wurde. Sollte unerwünschter Weise, bspw. wegen Überlastung der Messvorrichtung, der Ist-Zeitpunkt TI vom Soll-Zeitpunkt TS abweichen, wird der Vergleichsmesswert V und/oder Betriebsmesswert B mittels des angenäherten Messwertverlaufs L zum Soll-Zeitpunkt TS berechnet. Im in Fig. 2 dargestellten Beispiel wurde die Messung unabsichtlich verzögert, d.h. der Soll-Zeitpunkt TS liegt vor dem Ist-Zeitpunkt TI, weshalb fehlerhafterweise der Messwert M zum falschen Zeitpunkt erfasst würde. Zur Korrektur des Fehlers kann der Messwert M zum Soll-Zeitpunkt TS aus dem Messwertverlauf L berechnet werden.

Fig. 3 zeigt ein vereinfachtes Ablaufdiagramm gemäß dem Verfahren. Die Schritte S1 bis S7 müssen nicht notwendigerweise in der angegebenen Reihenfolge durchgeführt werden. Vielmehr kann der Fachmann eine zweckmäßige Reihenfolge der Schritte S1 bis S7 selbst festlegen.

In Schritt S1 wird der Sensor 1, 1a als Teil einer Messvorrichtung 2 zur Messung der Flüssigkeitsqualität bereitgestellt und die Messvorrichtung 2 wird relativ zur Flüssigkeit angeordnet.

In Schritt S2 wird der Sensor 1, 1a abwechselnd für die Dauer einer Einschaltphase TE aktiviert und für die Dauer einer Ausschaltphase TA deaktiviert. Insbesondere kann der Sensor 1, 1a zumindest zeitweise periodisch aktiviert und deaktiviert werden.

In Schritt S3 wird mit dem Sensor 1, 1a während einer definierten Einschaltphase TED ein Messwert M als Vergleichsmesswert V erfasst, für welche definierte Einschaltphase TED sich der Vergleichsmesswert V um höchstens eine vorgegebene Differenz D von einem während einer vorherigen Einschaltphase TE erfassbaren Wert W der Messgröße G unterscheidet.

In Schritt S4 wird eine Referenzmessung RM zur Erfassung eines die Flüssigkeitsqualität repräsentierenden Messwerts M, als Referenzmesswert R ausgeführt. Abhängig von der Art der Erfassung des Referenzmesswerts M kann der Schritt S4 auch früher durchgeführt werden. Die Möglichkeit einer früheren Erfassung ist in Fig. 3 durch die strichliert dargestellten Schritte S4 angedeutet.

In Schritt S5 werden mit dem Sensor 1, 1a während weiterer Einschaltphasen TEW, welche auf die definierte Einschaltphase TED folgen, laufend Messwerte M als Betriebsmesswerte B erfasst.

Die Schritte S2 bis S5 werden bevorzugt mit der gleichen Flüssigkeit durchgeführt.

In Schritt S6 wird eine Berechnungsvorschrift festgelegt, welche den Vergleichsmesswert V dem Referenzmesswert R zuordnet. Der Schritt S6 kann ausgeführt werden, sobald der Vergleichsmesswert V und der Referenzmesswert R bekannt sind, insbesondere vor dem Schritt S5.

In Schritt S7 wird mittels der Berechnungsvorschrift aus jedem Betriebsmesswert B ein finaler Messwert F zur Messung der Qualität ermittelt.

Es ist offensichtlich, dass einige Schritte des Ablaufs gleichzeitig ausgeführt werden. Zumindest die Schritte S3 und S5 bis S7 werden während der Ausführung des Schritts S2 durchgeführt.

Fig. 4 zeigt ein detaillierteres Ablaufdiagramm gemäß dem Verfahren. Auch in diesem Beispiel müssen die angegebenen Schritte S1 bis S10 nicht notwendigerweise in der angegebenen Reihenfolge durchgeführt werden. Die Schritte S1 bis S7 gemäß Fig. 4 entsprechen den Schritten S1 bis S7 gemäß Fig. 3 und werden daher in Zusammenhang mit Fig. 4 nicht erneut beschrieben.

Der Schritt S3 kann einen Schritt S3a oder einen Schritt S3b aufweisen. In Schritt S3a wird eine Einschaltphase TE zumindest dann als definierte Einschaltphase TED festgelegt, wenn eine festgelegte Anzahl von vorangehenden Einschaltphasen TE auf eine im Vergleich zur Dauer der Ausschaltphase TA verlängerte Ausschaltphase TA oder eine erstmalige Aktivierung des Sensors 1, 1a folgen. In Schritt S3b wird eine Einschaltphase TE zumindest durch folgendes als definierte Einschaltphase TED festgelegt: Es werden Messwerte M mit dem Sensor 1, 1a in mehreren aufeinander folgenden Einschaltphasen TE erfasst. Der zuletzt erfasste Messwert M wird mit dem davor erfassten Messwert M verglichen. Wenn die Abweichung der beiden verglichenen Messwerte M voneinander einen vorab definierten Schwellenwert unterschreitet, wird die Einschaltphase TE für den letzten Messwert M als die definierte Einschaltphase TED festgelegt. Darüber hinaus kann die definierte Einschaltphase TED auch auf andere Weise festgelegt werden.

Der Schritt S4 kann einen Schritt S4a nicht gemäß dem Verfahren oder einen Schritt S4b gemäß dem Verfahren aufweisen. In Schritt S4a wird eine Flüssigkeitsprobe von der Flüssigkeit entnommen und der Referenzmesswert R der entnommenen Flüssigkeitsprobe wird in einer zur Messvorrichtung 2 externen Vorrichtung erfasst. In Schritt S4b wird die definierte Einschaltphase TED oder eine Einschaltphase TEV zwischen der definierten Einschaltphase TED und den weiteren Einschaltphasen TEW, die der Erfassung der Betriebsmesswerte B dienen, verlängert. Während der verlängerten Einschaltphase TEV wird der Referenzmesswert R mit dem Sensor 1, 1a erfasst.

In den Schritten S3 und/oder S5 kann zudem vorgesehen sein, dass der Vergleichsmesswert V und/oder der Betriebsmesswert B durch Messen des Vergleichsmesswerts V und/oder des Betriebsmesswerts B zu einem festgelegten Soll-Zeitpunkt TS innerhalb der Einschaltphase TE, insbesondere am Ende der Einschaltphase TEE, erfasst werden.

Zudem kann in den Schritten S3 und/oder S5 vorgesehen sein, dass der Vergleichsmesswert V und/oder der Betriebsmesswert B durch folgendes erfasst werden:

- Messen mit dem Sensor 1, 1a zu mehreren Zeitpunkten TA innerhalb der dem Vergleichsmesswert V und/oder Betriebsmesswert B zugeordneten Einschaltphase TE, um mehrere Abtastwerte A zu erhalten;

- Berechnen eines an die Abtastwerte A angenäherten Messwertverlaufs L innerhalb der Einschaltphase TE;

- Messen zu welchem Ist-Zeitpunkt TI der für die Messung zu einem definierten Soll-Zeitpunkt TS vorgesehene Vergleichsmesswert V und/oder Betriebsmesswert B tatsächlich gemessen wurde; und

- im Falle einer Abweichung des Ist-Zeitpunkts TI vom Soll-Zeitpunkt TS, Berechnen des Vergleichsmesswerts V und/oder Betriebsmesswerts B zum Soll-Zeitpunkt TS mittels des angenäherten Messwertverlaufs L.

In Schritt S6 kann zudem vorgesehen sein, dass die Berechnungsvorschrift als Berechnen eines konstanten Faktors Q, welcher aus dem Quotienten zwischen dem Vergleichsmesswert V und dem Referenzmesswert R gebildet wird, festgelegt wird.

In Schritt S7 kann zudem vorgesehen sein, dass der finale Messwert F aus jedem Betriebsmesswert B durch Dividieren des Betriebsmesswerts B durch den berechneten konstanten Faktor Q ermittelt wird.

In Schritt S8 werden die Schritte S2 bis S7 erneut durchgeführt, wenn mehrere miteinander verglichene Betriebsmesswerte B oder mehrere miteinander verglichene finale Messwerte F um mehr als einen vorab definierten Schwellenwert voneinander abweichen.

In Schritt S9 werden zumindest die Schritte S2, S3 und S5 bis S7 erneut durchgeführt, wenn die Dauer der Einschaltphase TE und/oder die Dauer der Ausschaltphase TA um mehr als einen vorab definierten Schwellenwert geändert wurde.

In Schritt S10 wird eine Flüssigkeitspumpe 3 der Messvorrichtung 2, welche Flüssigkeitspumpe 3 die Flüssigkeit über den Sensor 1, 1a leitet, bereits vor dem Sensor (1, 1a) aktiviert, beispielsweise eine Minute bis zwei Minuten vor dem Schritt S2) aktiviert, insbesondere für einen Zeitraum welcher ausreichend ist, die Flüssigkeit dem Sensor (1, 1a) zuzuführen vor dem Sensor (1, 1a) aktiviert.

Fig. 5 zeigt ein vereinfachtes Blockschaltbild einer Messvorrichtung 2 zum stromsparenden fortlaufenden Messen FM einer Qualität einer Flüssigkeit, insbesondere von Wasser. Die Messvorrichtung 2 weist zumindest einen Sensor 1, 1a, einen Speicher 4 für elektrische Energie und eine Steuerung 5 auf. Die Steuerung 5 ist zur Durchführung bzw. zur Steuerung des Verfahrens ausgebildet. Zudem kann eine Flüssigkeitspumpe 3 vorgesehen sein. Die Steuerung 5 ist mit dem Sensor 1, 1a, dem Speicher 4 für elektrische Energie und der Flüssigkeitspumpe 3 verbunden. Zudem ist der Speicher 4 für elektrische Energie mit dem Sensor 1, 1a und der Flüssigkeitspumpe 3 verbunden. Die Messvorrichtung 2 kann zudem einen Datenspeicher 8, eine Eingabevorrichtung 6 zur Eingabe von Parametern, bspw. einem Referenzwert R, und eine Ausgabevorrichtung 7 zumindest für die finalen Messwerte F aufweisen. Zudem kann die Ausgabevorrichtung 7 eine Benachrichtigung ausgeben, wenn mehrere miteinander verglichene Betriebsmesswerte B oder mehrere miteinander verglichene finale Messwerte F um mehr als einen vorab definierten Schwellenwert voneinander abweichen.

**Patentansprüche**

1. Verfahren zum stromsparenden fortlaufenden Messen (FM) einer Qualität einer Flüssigkeit, insbesondere Wasser, mit einem Sensor (1, 1a) zur Erfassung einer Messgröße (G), wobei:

   S1) der Sensor (1, 1a) als Teil einer Messvorrichtung (2) zur Messung der Flüssigkeitsqualität bereitgestellt und die Messvorrichtung (2) relativ zur Flüssigkeit angeordnet wird;
   S2) der Sensor (1, 1a) abwechselnd für die Dauer einer Einschaltphase (TE) aktiviert und für die Dauer einer Ausschaltphase (TA) deaktiviert wird;
   S3) eine Einschaltphase (TE) in einem eingeschwungenen Zustand des Sensors (1, 1a) als definierte Einschaltphase (TED) festgelegt wird und mit dem Sensor (1, 1a) während der definierten Einschaltphase (TED) ein Messwert (M) als Vergleichsmesswert (V) erfasst wird, wobei der eingeschwungene Zustand erreicht ist, wenn sich ein Wert (W) der Messgröße (G) um höchstens eine vorgegebene Differenz (D) von einem Wert (W) der Messgröße (G) während einer vorherigen Einschaltphase (TE) unterscheidet;
   S4) eine Referenzmessung (RM) zur Erfassung eines die Flüssigkeitsqualität repräsentierenden Messwerts (M), als Referenzmesswert (R) ausgeführt wird;
   S5) mit dem Sensor (1, 1a) während weiterer Einschaltphasen (TEW), welche auf die definierte Einschaltphase (TED) folgen, laufend Messwerte (M) als Betriebsmesswerte (B) erfasst werden;
   S6) eine Berechnungsvorschrift festgelegt wird, welche den Vergleichsmesswert (V) dem Referenzmesswert (R) zuordnet; und
   S7) mittels der Berechnungsvorschrift aus jedem Betriebsmesswert (B) ein finaler Messwert (F) zur Messung der Qualität ermittelt wird; **dadurch gekennzeichnet, dass**
   für die Ausführung der Referenzmessung (RM)
   S4b) die definierte Einschaltphase (TED) oder eine Einschaltphase (TEV) zwischen der definierten Einschaltphase (TED) und den weiteren Einschaltphasen (TEW) zur Erfassung der Betriebsmesswerte (B) gegenüber den vorangegangenen Einschaltphasen (TE) verlängert wird, und während der verlängerten Einschaltphase (TEV) der Referenzmesswert (R) mit dem Sensor (1, 1a) erfasst wird.

2. Verfahren nach Anspruch 1, **dadurch gekennzeichnet, dass** der Sensor (1, 1a) zumindest zeitweise periodisch aktiviert und deaktiviert wird.

3. Verfahren nach Anspruch 1 oder 2, **dadurch gekennzeichnet, dass**
   S3a) eine Einschaltphase (TE) zumindest dann als definierte Einschaltphase (TED) festgelegt wird, wenn eine festgelegte Anzahl von vorangehenden Einschaltphasen (TE) auf eine im Vergleich zur Dauer der Ausschaltphase (TA) verlängerte Ausschaltphase (TA) oder eine erstmalige Aktivierung des Sensors (1, 1a) folgen.

4. Verfahren nach Anspruch 1 oder 2, **dadurch gekennzeichnet, dass**
   S3b) eine Einschaltphase (TE) zumindest durch folgendes als definierte Einschaltphase (TED) festgelegt wird:

   - Erfassen von Messwerten (M) mit dem Sensor (1, 1a) in mehreren aufeinander folgenden Einschaltphasen (TE);
   - Vergleichen des zuletzt erfassten Messwerts (M) mit dem davor erfassten Messwert (M); und
   - im Falle einer einen vorab definierten Schwellenwert unterschreitenden Abweichung der beiden verglichenen Messwerte (M) voneinander, Festlegen der Einschaltphase (TE) für den letzten Messwert (M) als die definierte Einschaltphase (TED).

5. Verfahren nach einem der Ansprüche 1 bis 4, **dadurch gekennzeichnet, dass** der Vergleichsmesswert (V) und/oder der Betriebsmesswert (B) durch Messen des Vergleichsmesswerts (V) und/oder des Betriebsmesswerts (B) zu einem festgelegten Soll-Zeitpunkt (TS) innerhalb der Einschaltphase (TE), insbesondere am Ende der Einschaltphase (TEE), erfasst werden.

6. Verfahren nach einem der Ansprüche 1 bis 5, **dadurch gekennzeichnet, dass** der Vergleichsmesswert (V) und/oder der Betriebsmesswert (B) durch folgendes erfasst werden:

   - Messen mit dem Sensor (1, 1a) zu mehreren Zeitpunkten (TA) innerhalb der dem Vergleichsmesswert (V) und/oder Betriebsmesswert (B) zugeordneten Einschaltphase (TE), um mehrere Abtastwerte (A) zu erhalten;
   - Berechnen eines an die Abtastwerte (A) angenäherten Messwertverlaufs (L) innerhalb der Einschaltphase (TE);
- Messen zu welchem Ist-Zeitpunkt (TI) der für die Messung zu einem definierten Soll-Zeitpunkt (TS) vorgesehene Vergleichsmesswert (V) und/oder Betriebsmesswert (B) tatsächlich gemessen wurde; und
- im Falle einer Abweichung des Ist-Zeitpunkts (TI) vom Soll-Zeitpunkt (TS), Berechnen des Vergleichsmesswerts (V) und/oder Betriebsmesswerts (B) zum Soll-Zeitpunkt (TS) mittels des angenäherten Messwertverlaufs (L).

7. Verfahren nach einem der Ansprüche 1 bis 6, **dadurch gekennzeichnet, dass**

- die Berechnungsvorschrift als Berechnen eines konstanten Faktors (Q), welcher aus dem Quotienten zwischen dem Vergleichsmesswert (V) und dem Referenzmesswert (R) gebildet wird, festgelegt wird und
- der finale Messwert (F) aus jedem Betriebsmesswert (B) durch Dividieren des Betriebsmesswerts (B) durch den berechneten konstanten Faktor (Q) ermittelt wird.

8. Verfahren nach einem der Ansprüche 1 bis 7, **dadurch gekennzeichnet, dass**
S8) die Schritte S2) bis S7) erneut durchgeführt werden, wenn mehrere miteinander verglichene Betriebsmesswerte (B) oder mehrere miteinander verglichene finale Messwerte (F) um mehr als einen vorab definierten Schwellenwert voneinander abweichen.

9. Verfahren nach einem der Ansprüche 1 bis 8, **dadurch gekennzeichnet, dass**
S9) zumindest die Schritte S2), S3) und S5) bis S7) erneut durchgeführt werden, wenn die Dauer der Einschaltphase (TE) und/oder die Dauer der Ausschaltphase (TA) um mehr als einen vorab definierten Schwellenwert geändert wurde.

10. Verfahren nach einem der Ansprüche 1 bis 9, **dadurch gekennzeichnet, dass**
S10) eine Flüssigkeitspumpe (3) der Messvorrichtung (2), welche Flüssigkeitspumpe (3) die Flüssigkeit über den Sensor (1, 1a) leitet, bereits vor dem Sensor (1, 1a) aktiviert wird, beispielsweise eine Minute bis zwei Minuten vor dem Schritt S2) aktiviert wird, insbesondere für einen Zeitraum welcher ausreichend ist, die Flüssigkeit dem Sensor (1, 1a) zuzuführen vor dem Sensor (1, 1a) aktiviert wird.

11. Verfahren nach einem der Ansprüche 1 bis 10, **dadurch gekennzeichnet, dass** als Sensor (1, 1a) ein Sensor (1a) zur Messung der Konzentration eines Desinfektionsmittels, insbesondere zur Messung einer Chlorkonzentration, oder zur Messung eines pH-Werts, verwendet wird.

12. Messvorrichtung (2) zum stromsparenden fortlaufenden Messen (FM) einer Qualität einer Flüssigkeit, insbesondere von Wasser, aufweisend zumindest einen Sensor (1, 1a), einen Speicher (4) für elektrische Energie und eine Steuerung (5), **dadurch gekennzeichnet, dass** die Steuerung (5) zur Durchführung eines Verfahrens gemäß den Ansprüchen 1 bis 11 ausgebildet ist.

13. Messvorrichtung (2) nach Anspruch 12, **dadurch gekennzeichnet, dass** der Sensor (1, 1a) zur Messung der Konzentration eines Desinfektionsmittels, insbesondere zur Messung einer Chlorkonzentration, oder zur Messung eines pH-Werts ausgebildet ist.

**Claims**

1. Method for energy-saving continuous measuring (FM) of the quality of a fluid,
in particular water, with a sensor (1, 1a) for capturing a measured quantity (G), wherein:

S1) the sensor (1, 1a) is provided as part of a measuring device (2) for measuring the liquid quality and the measuring device (2) is arranged relative to the fluid;
S2) the sensor (1, 1a) is alternately activated for the duration of a switch-on phase (TE) and deactivated for the duration of a switch-off phase (TA);
S3) a switch-on phase (TE) in a steady state of the sensor (1, 1a) is defined as a defined switch-on phase (TED) and a measured value (M) is determined with the sensor (1, 1a) during the defined switch-on phase (TED) as a comparative measured value(V) is captured, the steady state being reached when a value (W) of the measured quantity (G) differs by at most a predetermined difference (D) from a value (W) of the measured quantity (G) during a previous switch-on phase (TE);
S4) a reference measurement (RM) is performed to capture a measured value (M) representing the liquid quality as a reference measured value (R);
S5) with the sensor (1, 1a) during further switch-on phases (TEW), which follow the defined switch-on phase (TED), measured values (M) are continuously captured as operating measured values (B);
S6) a calculation method is defined which assigns the comparative measured value (V) to the reference measured value (R); and

S7) a final measured value (F) for measuring the quality is determined from each operating measured value (B) by means of the calculation method; **characterized in that**

for carrying out the reference measurement (RM) S4b) the defined switch-on phase (TED) or a switch-on phase (TEV) between the defined switch-on phase (TED) and the further switch-on phases (TEW) is extended compared to the previous switch-on phases (TE) in order to capture the operating measured values (B), and during the extended switch-on phase (TEV) the reference measured value (R) is captured with the sensor (1, 1a).

2. Method according to claim 1, **characterized in that** the sensor (1, 1a) is activated and deactivated at least periodically.

3. Method according to claim 1 or 2, **characterized in that** S3a) a switch-on phase (TE) is defined as a defined switch-on phase (TED) at least if a defined number of preceding switch-on phases (TE) follow a switch-off phase (TA) that is longer than the duration of the switch-off phase (TA) or if the sensor (1, 1a) is activated for the first time.

4. Method according to claim 1 or 2, **characterized in that** S3b) a switch-on phase (TE) is defined as a defined switch-on phase (TED) by at least the following:

   - Capturing measured values (M) with the sensor (1, 1a) in several successive switch-on phases (TE);
   - Compare the last measured value (M) captured with the previous measured value (M); and
   - in the event of a deviation of the two compared measured values (M) from each other that does not exceed a predefined threshold value, setting the switch-on phase (TE) for the last measured value (M) as the defined switch-on phase (TED).

5. Method according to one of claims 1 to 4, **characterized in that** the comparative measured value (V) and/or the operating measured value (B) are captured by measuring the comparative measured value (V) and/or the operating measured value (B) at a fixed target-time (TS) within the switch-on phase (TE), in particular at the end of the switch-on phase (TEE).

6. Method according to one of claims 1 to 5, **characterized in that** the comparative measured value (V) and/or the operating measured value (B) are captured by the following:

   - Measuring with the sensor (1, 1a) at several points in time (TA) within the switch-on phase (TE) assigned to the comparative measured value (V) and/or operating measured value (B) in order to obtain several scanning values (A);
   - Calculation of a measured value progression (L) approximated to the scanning values (A) within the switch-on phase (TE);
   - Measuring the actual-time (TI) at which the comparative measured value (V) and/or operating measured value (B) intended for measurement at a defined target-time (TS) was actually measured; and
   - in the event of a deviation of the actual-time (TI) from the target-time (TS), calculation of the comparative measured value (V) and/or operating measured value (B) to the target-time (TS) using the approximated measured value progression (L).

7. Method according to any one of claims 1 to 6, **characterized in that**

   - the calculation method is defined as calculating a constant factor (Q), which formed from the quotient between the comparative measured value (V) and the reference measured value (R), and
   - the final measured value (F) is determined from each operating measured value (B) by dividing the operating measured value (B) by the calculated constant factor (Q).

8. Method according to any one of claims 1 to 7, **characterized in that** S8) steps S2) to S7) are performed again if several operating measured values (B) compared with each other or several final measured values (F) compared with each other deviate from each other by more than a predefined threshold value.

9. Method according to any one of claims 1 to 8, **characterized in that** S9) at least steps S2), S3) and S5) to S7) are performed again if the duration of the switch-on phase (TE) and/or the duration of the switch-off phase (TA) has been changed by more than a predefined threshold value.

10. Method according to any one of claims 1 to 9, **characterized in that** S10) a fluid pump (3) of the measuring device (2), whereby fluid pump (3) directs the fluid over the sensor (1, 1a), is already activated before the sensor (1, 1a), already before the sensor (1, 1a) is activated, for example, one minute to two minutes before step S2) is activated, in particular for a period of time which is sufficient to supply the fluid to the sensor (1, 1a) before the sensor (1, 1a) is activated.

**11.** Method according to any one of claims 1 to 10, **characterized in that** the sensor (1, 1a) used is a sensor (1a) for measuring the concentration of a disinfectant, in particular for measuring a chlorine concentration, or for measuring a pH value.

**12.** Measuring device (2) for the energy-saving continuous measuring (FM) of a quality of a fluid, in particular of water, comprising at least one sensor (1, 1a), a storage (4) for electrical energy and a controlling unit (5), **characterized in that** the controlling unit (5) is designed to carry out a method according to claims 1 to 11.

**13.** Measuring device (2) according to claim 12, **characterized in that** the sensor (1, 1a) is designed for measuring the concentration of a disinfectant, in particular for measuring a chlorine concentration, or for measuring a pH value.

**Revendications**

**1.** Procédé de mesure en continu à faible de consommation de courant (FM) d'une qualité d'un liquide, plus particulièrement de l'eau, avec un capteur (1, 1a) pour la mesure d'une grandeur de mesure (G), dans lequel :

S1) le capteur (1, 1a) est conçu comme une partie d'un dispositif de mesure (2) pour la mesure de la qualité d'un liquide et le dispositif de mesure (2) est disposé par rapport au liquide ;
S2) le capteur (1, 1a) est alternativement activé pendant la durée d'une phase d'activation (TE) et désactivé pendant la durée d'une phase de désactivation (TA) ;
S3) une phase d'activation (TE) dans un état stationnaire du capteur (1, 1a) est déterminée comme phase d'activation définie (TED) et le capteur (1, 1a) permet de mesurer, pendant la phase d'activation définie (TED), une valeur de mesure (M) en tant valeur de mesure de comparaison (V), dans lequel l'état stationnaire est atteint lorsqu'une valeur (W) de la grandeur de mesure (G) est différente de maximum une différence prédéterminée (D) par rapport à une valeur (W) de la grandeur de mesure (G) pendant une phase d'activation (TE) précédente ;
S4) une mesure de référence (RM) est effectuée afin de mesurer une valeur de mesure (M) représentant la qualité du liquide, en tant que valeur de mesure de référence (R) ;
S5) le capteur (1, 1a) mesure, pendant d'autres phases d'activation (TEW), qui suivent la phase d'activation définie (TED), en continu des valeurs de mesure (M) en tant que valeurs de mesure de service (B) ;

S6) une instruction de calcul est déterminée, qui attribue la valeur de mesure de comparaison (V) à la valeur de mesure de référence (R) ;
S7) l'instruction de calcul permet de déterminer, à partir de chaque valeur de mesure de service (B), une valeur de mesure finale (F) pour la mesure de la qualité ;
**caractérisé en ce que**, pour l'exécution de la mesure de référence (RM)
S4b) la phase d'activation définie (TED) ou une phase d'activation (TEV), entre la phase d'activation définie (TED) et les autres phases d'activation (TEW), est prolongée par rapport aux phases d'activation (TE) précédentes, pour la mesure des valeurs de mesure de service (B) et, pendant la phase d'activation prolongée (TEV), la valeur de mesure de référence (R) est mesurée avec le capteur (1, 1a).

**2.** Procédé selon la revendication 1, **caractérisé en ce que** le capteur (1, 1a) est activé et désactivé au moins temporairement de manière périodique.

**3.** Procédé selon la revendication 1 ou 2, **caractérisé en ce que**
S3a) une phase d'activation (TE) est déterminée en tant que phase d'activation définie (TED) au moins lorsqu'un nombre prédéterminé de phases d'activation (TE) précédentes suivent une phase de désactivation (TA) prolongée par rapport à la durée de la phase de désactivation (TA) ou une première activation du capteur (1, 1a).

**4.** Procédé selon la revendication 1 ou 2, **caractérisé en ce que**
S3b) une phase d'activation (TE) est déterminée au moins par ce qui suit en tant que phase d'activation définie (TED) :

- mesure de valeurs de mesure (M) avec le capteur (1, 1a) dans plusieurs phases d'activation (TE) successives ;
- comparaison des dernières valeurs de mesure (M) mesurées avec la valeur de mesure (M) mesurée précédemment ; et
- dans le cas d'un écart, en dessous d'une valeur seuil définie préalablement, des deux valeurs de mesure (M) comparées, définition de la phase d'activation (TE) pour la dernière valeur de mesure (M) en tant que phase d'activation définie (TED).

**5.** Procédé selon l'une des revendications 1 à 4, **caractérisé en ce que** la valeur de mesure de comparaison (V) et/ou la valeur de mesure de service (B) sont mesurées grâce à la mesure de la valeur de mesure de comparaison (V) de la valeur de mesure de service (B) à un instant de consigne (TS) à l'intérieur de la phase d'activation (TE), plus particulièrement à la fin de la phase d'activation (TEE).

6. Procédé selon l'une des revendications 1 à 5, **caractérisé en ce que** la valeur de mesure de comparaison (V) et/ou la valeur de mesure de service (B) sont mesurées grâce à ce qui suit :

> - mesure, avec le capteur (1, 1a), à plusieurs instants (TA) à l'intérieur de la phase d'activation (TE) correspondant à la valeur de mesure de comparaison (V) et/ou à la valeur de mesure de service (B), afin d'obtenir plusieurs valeurs de balayage (A) ;
> - calcul d'un tracé de valeurs de mesure (L) approximant les valeurs de balayage (A) à l'intérieur de la phase d'activation (TE) ;
> - mesure à quel instant effectif (TI) la valeur de mesure de comparaison (V) et/ou la valeur de mesure de service (B), prévue pour la mesure à un instant de consigne (TS) défini, a effectivement été mesurée ; et
> - dans le cas d'un écart entre l'instant effectif (TI) et l'instant de consigne (TS), calcul de a valeur de mesure de comparaison (V) et/ou de la valeur de mesure de service (B) à l'instant de consigne (TS) au moyen du tracé de valeurs de mesure (L) approximé.

7. Procédé selon l'une des revendications 1 à 6, **caractérisé en ce que**

> - l'instruction de calcul est définie comme le calcul d'un facteur constant (Q), qui est formé à partir du rapport entre la valeur de mesure de comparaison (V) et la valeur de mesure de référence (R) et
> - la valeur de mesure finale (F) est déterminée à partir de chaque valeur de mesure de service (B) par la division de la valeur de mesure de service (B) par le facteur constant (Q) calculé.

8. Procédé selon l'une des revendications 1 à 7, **caractérisé en ce que**
S8) les étapes S2 à S7) sont exécutées à nouveau lorsque plusieurs valeurs de mesure de service (B) comparées entre elles ou plusieurs valeurs de mesure finales (F) comparées entre elles sont différentes d'une valeur supérieure à une valeur seuil prédéfinie.

9. Procédé selon l'une des revendications 1 à 8, **caractérisé en ce que**
S9) au moins les étapes S2), S3 et S5) à S7), sont exécutées à nouveau lorsque la durée de la phase d'activation (TE) et/ou la durée de la phase de désactivation (TA) ont été modifiées d'une valeur supérieure à la une valeur seuil prédéfinie.

10. Procédé selon l'une des revendications 1 à 9, **caractérisé en ce que**
S10) une pompe à liquide (3) du dispositif de mesure (2), cette pompe à liquide (3) conduisant le liquide à travers le capteur (1, 1a), est activée avant le capteur (1, 1a), par exemple activée une minute à deux minutes avant l'étape S2), plus particulièrement activée avant le capteur (1, 1a) pendant une période qui est suffisante pour conduire le liquide vers le capteur (1, 1a).

11. Procédé selon l'une des revendications 1 à 10, **caractérisé en ce que** le capteur (1, 1a) utilisé est un capteur (1a) pour la mesure de la concentration d'un produit désinfectant, plus particulièrement pour la mesure d'une concentration en chlore ou pour la mesure d'une valeur de pH.

12. Dispositif de mesure (2) pour la mesure en continu à faible de consommation de courant (FM) d'une qualité d'un liquide, plus particulièrement de l'eau, comprenant au moins un capteur (1, 1a), un accumulateur (4) pour l'énergie électrique et une commande (5), **caractérisé en ce que** la commande (5) est conçue pour l'exécution d'un procédé selon les revendications 1 à 11.

13. Dispositif de mesure (2) selon la revendication 12, **caractérisé en ce que** le capteur (1, 1a) est conçu pour la mesure de la concentration d'un produit désinfectant, plus particulièrement pour la mesure d'une concentration en chlore, ou pour la mesure d'une valeur de pH.

Fig. 1

Fig. 2

Fig. 4

Fig. 3

Fig. 5

16

**IN DER BESCHREIBUNG AUFGEFÜHRTE DOKUMENTE**

**In der Beschreibung aufgeführte Patentdokumente**

- AT 520515 A1
- EP 3194908 B1
- US 20200340968 A1
- JP S59162444 A
- US 4464230 A
- US 2009068754 A1